# EUROPEAN PATENT APPLICATION

(11) **EP 4 407 521 A1**
(43) Date of publication of application: **31.07.2024**
(21) Application number: 23153925.5
(22) Date of filing: 30.01.2023
(51) Int. Cl.: G06N 3/0475, G06N 3/092, G06N 3/094, G06N 20/00

(54) **SYSTEMS AND METHODS FOR CONSENT REVOCATION AND TRAINING OF MACHINE LEARNING MODELS**

(71) Applicant: Koninklijke Philips N.V., 5656 AG Eindhoven (NL)
(72) Inventor: RAO KRUPASHANKAR RAO, Raghavendra, 5656 AG Eindhoven (NL); KAKATHKAR, Varsha, 5656 AG Eindhoven (NL); VINCHU SUBRAMANIAN, Balakrishnan, 5656 AG Eindhoven (NL); GOYAL, Shivani, 5656 AG Eindhoven (NL); MANDHAN, Sunil, 5656 AG Eindhoven (NL); SISODIA, Rajendra Singh, 5656 AG Eindhoven (NL)
(74) Representative: Philips Intellectual Property & Standards

(57) **Abstract**

The present disclosure relates to methods and systems for training machine learning models in a data environment where the training data is subject to deletion and/or quarantine. In particular applications, the training data may include medical information for which an individual may revoke consent to use and/or access at any time. As described herein, the methods and systems involve: receiving a consent revocation notice, wherein the notice identifies a first subset of data from a comprehensive dataset; estimating an impact of the first subset of data on the machine learning model; generating a synthetic dataset corresponding to the first subset of data; generating a training dataset comprising the comprehensive dataset and the synthetic dataset; and training the machine learning model on the training dataset.

## Description

### FIELD OF THE DISCLOSURE

The present disclosure relates generally to the training of machine learning (ML) models, and more specifically to systems and methods of training ML models in data environments subject to consent revocation.

### BACKGROUND

Data privacy is a concern for many individuals and industries. Especially in the healthcare industry, mishandling of sensitive medical data can result in significant penalties even when the mishandling is not done maliciously or with the intent to violate applicable laws or regulations. On the other hand, access to such data can be a powerful tool for advancements in machine learning (ML) applications. Oftentimes, a ML model that is used to generate predictions (e.g., risk of readmission, likelihood of ventilation, discharge status, etc.) is only as good as the dataset used to train the model. Thus, a larger data set can result in more accurate findings. Nevertheless, individuals for whom medical data has been collected retain the power to revoke consent over the use of their medical data, which can affect the training and ultimately the accuracy of trained ML models.

### SUMMARY OF THE DISCLOSURE

According to an embodiment of the present disclosure, a method of training a machine learning model is provided. The method comprises: receiving a consent revocation notice, wherein the notice identifies a first subset of data from a comprehensive dataset; estimating an impact of the first subset of data on the machine learning model; generating a synthetic dataset corresponding to the first subset of data; generating a training dataset comprising the comprehensive dataset and the synthetic dataset; and training the machine learning model on the training dataset.

In an aspect, the training dataset does not include the first subset of data.

In an aspect, the consent revocation notice identifies the first subset of data for which consent to use the first subset of data has been revoked.

In an aspect, the method further comprises: training the machine learning model on the comprehensive dataset before receiving a consent revocation notice, wherein the comprehensive dataset comprises at least the first subset of data.

In an aspect, generating the synthetic dataset includes: generating the synthetic dataset using a generative adversarial network, wherein the synthetic dataset has one or more data features; determining whether the one or more data features of the synthetic dataset match one or more data features of the first subset of data within a predetermined threshold; repeating the generation and determination steps until the one or more data features of the synthetic dataset match the one or more data features of the first subset of data within the predetermined threshold.

In an aspect, the one or more features of the synthetic dataset includes at least one of a dataset scale, a dataset shape, a dataset threshold, and a dataset distribution; and wherein the one or more features of the first subset of data includes at least one of a dataset scale, a dataset shape, a dataset threshold, and a dataset distribution.

In an aspect, the synthetic dataset is generated in response to the estimated impact of the first subset of data on the machine learning model.

In an aspect, the estimated impact comprises a count of one or more model parameters and/or predictions that are likely to change when the machine learning model is trained using the training dataset that does not include the first subset of data.

In an aspect, the estimated impact comprises a degree to which one or more model parameters and/or predictions are likely to change by when the machine learning model is trained using the training dataset that does not include the first subset of data.

According to another embodiment of the present disclosure, a system configured to train a machine learning model is provided. The system comprises: a database storing a comprehensive dataset of medical information of a plurality of patients; a consent management system configured to manage consent settings for the medical information of the plurality of patients; and one or more processors in communication with the database and the consent management system. In an aspect, the one or more processors are configured to: receive a consent revocation notice from the consent management system, wherein the notice identifies a first subset of data from the comprehensive dataset; estimate an impact of the first subset of data on the machine learning model; generate a synthetic dataset corresponding to the first subset of data; generate a training dataset comprising the comprehensive dataset and the synthetic dataset; and train the machine learning model on the training dataset.

In an aspect, the training dataset does not include the first subset of data.

In an aspect, the one or more processors are further configured to: train the machine learning model on the comprehensive dataset before receiving a consent revocation notice, wherein the comprehensive dataset comprises at least the first subset of data.

In an aspect, the one or more processors are configured to generate the synthetic dataset by: generating the synthetic dataset using a generative adversarial network, wherein the synthetic dataset has one or more data features; determining whether the one or more data features of the synthetic dataset matches one or more data features of the first subset of data within a predetermined threshold; and repeating the generation and determination steps until the one or more data features of the synthetic dataset matches the one or more data features of the first subset of data within the predetermined threshold.

In an aspect, the one or more features of the synthetic dataset includes at least one of a dataset scale, a dataset shape, a dataset threshold, and a dataset distribution; and wherein the one or more features of the first subset of data includes at least one of a dataset scale, a dataset shape, a dataset threshold, and a dataset distribution.

In an aspect, the synthetic dataset is generated in response to the estimated impact of the first subset of data on the machine learning model.

These and other aspects of the various embodiments will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a flowchart of a method for training a machine learning model illustrated according to aspects of the present disclosure.
FIG. 2A is a flowchart of certain steps of a method for training a machine learning model illustrated according to aspects of the present disclosure.
FIG. 2B is a flowchart of certain steps of a method for training a machine learning model illustrated according to aspects of the present disclosure.
FIG. 3 is a flow diagram of a system for training a machine learning model illustrated according to aspects of the present disclosure.
FIG. 4A is a schematic block diagram of a device used in the training of a machine learning model illustrated according to aspects of the present disclosure.
FIG. 4B is a block diagram of a portion of a device used in the training of a machine learning model illustrated according to aspects of the present disclosure.

### DETAILED DESCRIPTION OF EMBODIMENTS

Large datasets can be created to create and/or train machine learning (ML) models. In healthcare applications, widespread use of electronic healthcare records have enabled the curation of large datasets of patient information. As a result, a number of beneficial ML models have been developed that are trained on healthcare information from a plurality of patients (e.g., hundreds, thousands, and more). However, legislation and industry standards require that healthcare providers obtain consent to use and/or disclose sensitive healthcare information. Healthcare providers may utilize a consent management system to manage the access and storage of healthcare information based on the consent or lack of consent of the corresponding patient. However, because one or more patients may revoke consent connected with their medical records (or a portion thereof), there is a lack of continuity in the datasets used to train ML models. As a result, this lack of continuity creates instabilities in the trained ML models that impair accuracy and/or otherwise skew results of the ML models.

Accordingly, the present disclosure is directed to methods and systems adapted for use in data frameworks where datasets used to train one or more machine learning models are subject to consent revocation.

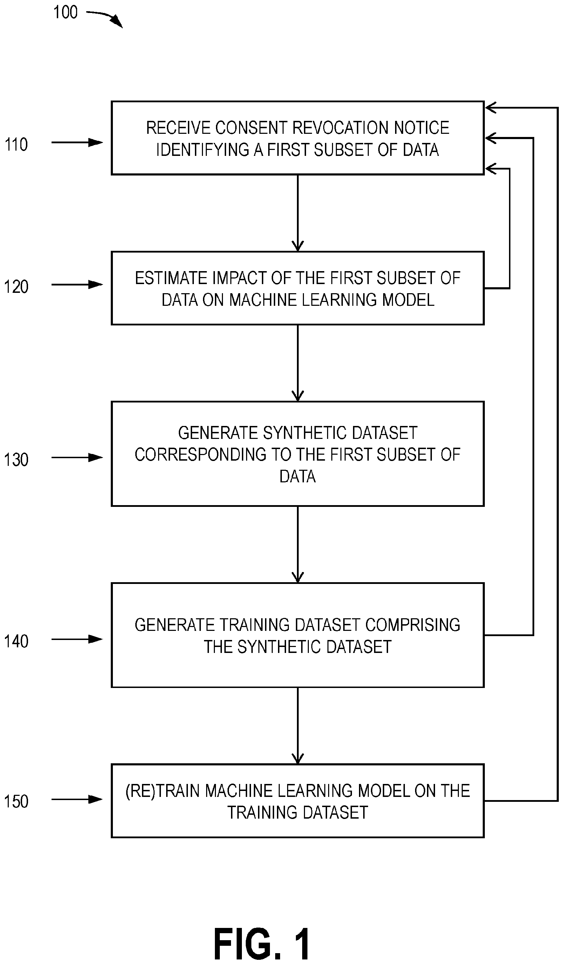

Turning to FIG. 1, a method 100 of training a machine learning model is illustrated according to aspects of the present disclosure. As discussed above, the method 100 may be a method of training a machine learning model in a data framework where datasets used to train the machine learning model are subject to consent revocation.

At a step 110, the method 100 includes receiving a consent revocation notice that identifies at least a first subset of data from among a comprehensive dataset. In embodiments, the consent revocation notice identifies information for which consent to use and/or disclose has been revoked. For example, if a first patient has electronic medical records stored in a database of a plurality of patients that is being managed by a healthcare provider, the first patient may revoke consent for the healthcare provider to use those electronic medical records for various purposes (including the training of machine learning models).

In embodiments, the comprehensive dataset can be a collection of medical records comprising medical information for a plurality of individuals. This medical information can include, but is not limited to, information such as identification information (e.g., date of birth, name, marital status, social security number, etc.), medical history (e.g., allergies, treatments, medical care, past and present diagnoses, habits such as diet, alcohol intake, exercise, etc.), medication information, family history, treatment history (e.g., complaints, history of illness, vital signs, physical exams, surgical history, immunization history), medical directives, lab results (e.g., lab results related to cells, tissues, body fluids, or imaging tests), consent forms, and the like.

In some embodiments, the comprehensive dataset includes medical records for multiple patients, including at least 10, or at least 100, or at least 1,000, or at least 10,000, or at least 100,000 patients.

In further embodiments, the first subset of data identified by the consent revocation notice includes medical records containing medical information for one individual. In still further embodiments, the first subset of data identified by the consent revocation notice includes medical records containing medical information for multiple individuals. Put another way, the consent revocation notice received in step 110 can identify a subset of data related to a plurality of patients for whom consent to use such data has been revoked. For instance, a parent and/or legal guardian may revoke consent for a healthcare provider to use and/or disclose medical information related to themselves, their spouse, and/or their children. As such, in embodiments, the step 110 can include receiving multiple consent revocation notice corresponding to each person, or can include receiving a single consent revocation notice corresponding to multiple individuals.

In embodiments, the consent revocation notice can include the first subset of data itself. That is, the consent revocation notice may include a subset of data including medical records that contain medical information for which consent has been revoked.

At a step 120, the method 100 includes estimating an impact of the first subset of data on a machine learning model. In embodiments, the machine learning model can be a model that was previously trained on a comprehensive dataset that comprises at least the first subset of data. Thus, in embodiments, the step 120 includes estimating the impact of removing the first subset of data from a comprehensive dataset used to train the machine learning model.

In embodiments, the impact of the first subset of data on the machine learning model may be estimated and/or quantified in one or more ways.

In some embodiments, the impact of the first subset of data on the machine learning model may be estimated and/or quantified by retraining the model without the revoked data points and measuring the changes in accuracy when validated on a validation dataset. As used herein, the validation dataset can be a dataset containing medical records and medical information for one or more actual and/or fictitious individuals that is mutually exclusive with the comprehensive dataset (i.e., does not include the medical records or the medical information of the comprehensive dataset).

In other embodiments, the impact of the first subset of data on the machine learning model may be estimated and/or quantified by using an established permutation-based method. For example, an influence function may be used to measure the dependence of the estimator on the value of one of the points in the sample.

In still other embodiments, the impact of the first subset of data on the machine learning model may be estimated and/or quantified by a game theory-based method to determine a dataset valuation metric. For example, a Data Shapley Algorithm may be used to determine the value of each training data point and/or dataset with respect to the machine learning algorithm and one or more performance metrics of the machine learning algorithm.

In yet other embodiments, the impact of the first subset of data on the machine learning model may be estimated and/or quantified by a data valuation method using reinforcement learning (DVRL). In a DVRL method, a data value estimator is used to estimate data values and select the most valuable samples to train a predictor model. Reinforcement learning (RL) may be used such that the supervision of the DVE is based on a reward that quantifies the model's performance on a validation dataset. In embodiments, the reward may guide the optimization of the DVE towards the action of optimal data valuation given a particular state and input samples.

In embodiments, if the impact of the revoked dataset on the performance of the machine learning model is below a predetermined threshold and/or within a predetermined tolerance level, then the method 100 may end. In other embodiments, the method 100 may repeat starting with step 110. In embodiments, if the impact of the revoked dataset on the performance of the machine learning model is below a predetermined threshold and/or within a predetermined tolerance level, a history of one or more consent revocation notices may be recorded, updated, or otherwise tracked. In such embodiments, the revoked dataset may comprise one or more datasets identified by one or more consent revocation notices. In embodiments, if the estimated impact of the revoked dataset on the performance of the machine learning model exceeds a predetermined threshold and/or exceeds a predetermined tolerance level, then the method 100 may proceed to the step 130.

At a step 130, the method 100 includes generating a synthetic dataset corresponding to the first subset of data. As used herein, the synthetic dataset can comprise simulated medical information that excludes the actual medical information contained in the first subset of data but, as an aggregate, mimics the first subset of data without compromising any legal and/or regulatory aspects surrounding the revoked dataset.

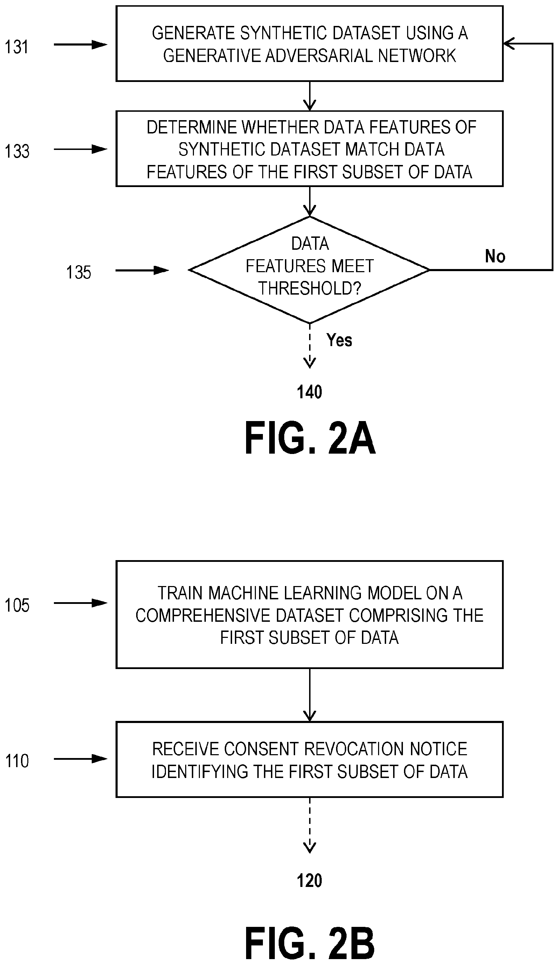

In embodiments, a deep-learning-based generative model may be used to generate the synthetic medical information based on one or more features of the first subset of data. In particular embodiments, as shown in FIG. 2A, the step 130 can include: in a step 131, generating the synthetic dataset using a deep-learning-based generative model, where the synthetic dataset has one or more data features; in a step 133, determining whether the one or more data features of the synthetic dataset matches one or more data features of the first subset of data within a predetermined threshold; and in a step 135, repeating the generation and determination steps until the one or more data features of the synthetic dataset matches the one or more data features of the first subset of data within the predetermined threshold.

In particular embodiments, the deep-learning-based generative model a generative adversarial network model. More particularly, according to the present disclosure, a GAN model can include a deep generative model 'G' and a discriminative model 'D'. In embodiments, the deep generative model 'G' may generate new data instances from random input and learns to generate data points that look more like the sample data point such that the discriminative model `D' fails to discriminate between the actual and the generated data point. In embodiments, the discriminative model 'D' may be trained on both real data and synthetic data (from the generative model 'G'), and can be configured to evaluate whether the input data is real or synthetic.

In embodiments, the one or more data features of the first subset of data may include at least one of a dataset scale, a dataset shape, a dataset threshold, a dataset distribution, and the like, including combinations thereof. In further embodiments, the one or more data features of the synthetic dataset may include at least one of a dataset scale, a dataset shape, a dataset threshold, a dataset distribution, and the like, including combinations thereof.

In embodiments, step 130 of the method 100 may further include resetting the history of the consent revocation notices. That is, after generating a synthetic dataset based on one or more revoked datasets, the method 100 can include clearing out the record of historical revoked datasets.

At a step 140, the method 100 includes generating a training dataset that comprises the synthetic dataset generated in step 130. In embodiments, the training dataset includes the comprehensive dataset, or a portion thereof. In particular embodiments, the training dataset includes the comprehensive dataset but does not include the first subset of data for which consent has been revoked. In some embodiments, the training dataset can include one or more synthetic datasets. For example, if multiple consent revocation notices were received, then distinct synthetic datasets may be generated for each subset of data and incorporated into the training dataset.

At a step 150, the method 100 includes training the machine learning model using the training dataset. In particular embodiments, the step 150 may not be performed every time a consent revocation notice is received. For example, if the impact of the consent revocation is not significant or if the cost of re-training the machine learning model is high, the method 100 may be repeated from step 110 to step 140 one or more times, wherein each time the training dataset is updated to include one or more synthetic datasets. Accordingly, in some embodiments, the step 150 includes training the machine learning model using a training dataset that comprises more than one synthetic dataset, wherein each synthetic dataset corresponds to a different subset of data for which consent was revoked (i.e., for which a consent revocation notice was received).

In further embodiments, the step 150 can include retraining the machine learning model using the training dataset. For example, as shown in FIG. 2B, the method 100 can include, in a step 105, training the machine learning model on a comprehensive dataset before receiving a consent revocation notice. Put another way, the machine learning model may be trained on the comprehensive dataset comprising one or more subsets of data that are subject to consent revocation. In embodiments, the machine learning model is trained on a comprehensive dataset comprising at least the first subset of data that is subsequently identified in a consent revocation notice.

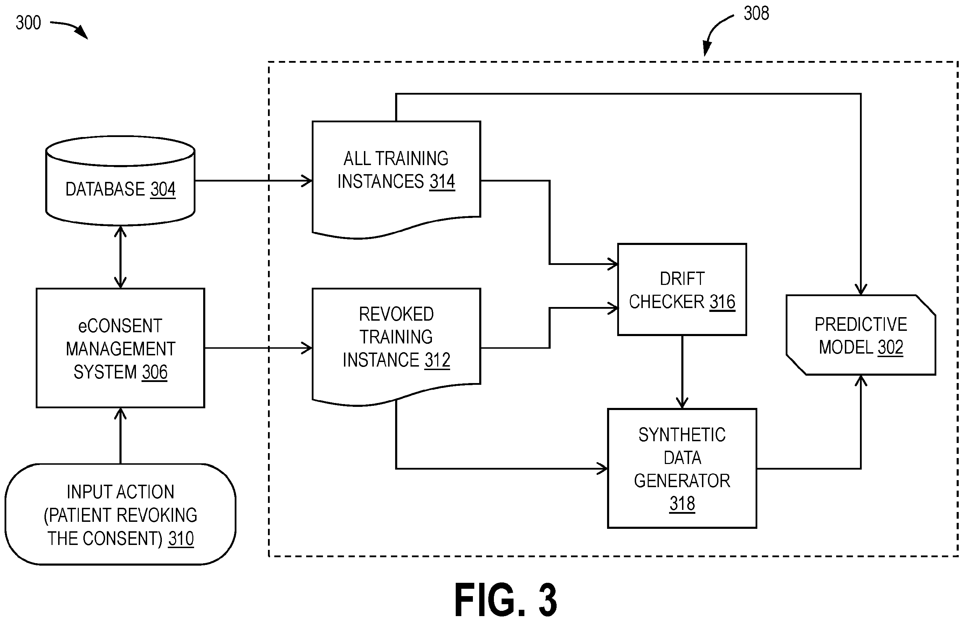

Also provided herein are systems for training a machine learning model in a data environment where training data is subject to removal (e.g., through consent revocation). For example, with reference to FIG. 3, a system 300 configured to train a machine learning model 302 is illustrated according to aspects of the present disclosure. As shown in FIG. 3, the system 300 may include at least a database 304, a consent management system 306, and/or one or more processors 308 configured to perform one or more steps of the methods described herein.

The database 304 may be a database storing a comprehensive dataset of medical information for a plurality of patients. For example, the comprehensive dataset can be a collection of medical records comprising medical information for a plurality of individuals. This medical information can include, but is not limited to, information such as identification information (e.g., date of birth, name, marital status, social security number, etc.), medical history (e.g., allergies, treatments, medical care, past and present diagnoses, habits such as diet, alcohol intake, exercise, etc.), medication information, family history, treatment history (e.g., complaints, history of illness, vital signs, physical exams, surgical history, immunization history), medical directives, lab results (e.g., lab results related to cells, tissues, body fluids, or imaging tests), consent forms, and the like. In particular embodiments, the comprehensive dataset includes medical records for multiple patients, including at least 10, or at least 100, or at least 1,000, or at least 10,000, or at least 100,000 patients.

The consent management system 306 can be, for example and without limitation, an eConsent Management System configured to manage one or more consent settings for the medical information of a plurality of patients. In embodiments, the consent management system 306 is configured to manage one or more consent settings for the medical information of the comprehensive dataset stored in the database 304. In particular embodiments, the consent management system 306 contains a process and/or set of policies for allowing individuals to determine what personal information they are willing to share, including what medical information they share. In specific embodiments, the consent management system 306 can enable a healthcare provider to take consent electronically from individuals (e.g., patients and/or participants, etc.). In further embodiments, the consent management system 306 can enable such individuals to revoke consent to access and/or use their information at any time.

In embodiments, the consent management system 306 may govern access to the data stored in the database 304, in order to, for example, delete and/or quarantine a subset of the stored data. That is, the consent management system 306 may receive an input action 310 revoking consent to a subset of the data stored in the database 304. The consent management system 306 may then generate a consent revocation notice identifying and/or including the revoked subset of data 312, which is communicated to the one or more processors 308.

Accordingly, in embodiments, the one or more processors 308 may be configured to receive a consent revocation notice identifying and/or including the revoked subset of data 312. The one or more processors 308 may be further configured to: estimate an impact of the first subset of data 312 on the machine learning model 302; generate a synthetic dataset corresponding to the first subset of data 312; generate a training dataset comprising a comprehensive dataset 314 and the synthetic dataset; and train the machine learning model 302 on the training dataset.

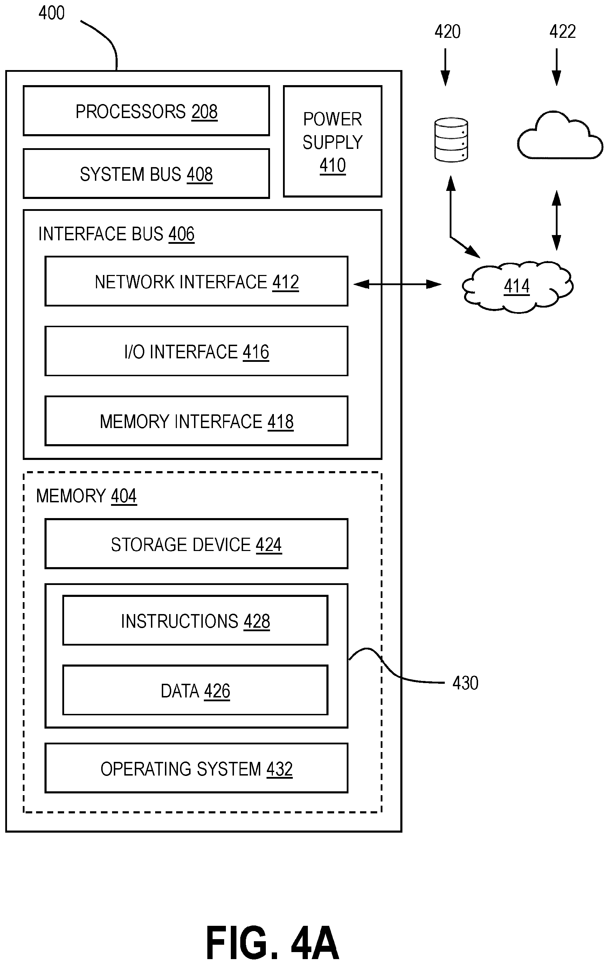

With reference to FIG. 4A, the one or more processors 308 may be embodied in a device 400, which may form part of the system 300 as discussed above. In particular, the system 300 may comprise a device 400 having one or more processors 208 configured to perform one or more steps of the methods described herein.

In the example of FIG. 4A, the device 400 can include the one or more processors 208, machine-readable memory 404, and an interface bus 406, all of which may be interconnected and/or communicate through a system bus 408 containing conductive circuit pathways through which instructions (e.g., machine-readable signals) may travel to effectuate communication, tasks, storage, and the like. The device 400 may be connected to a power source 410, which can include an internal power supply and/or an external power supply.

The one or more processors 208 may include a high-speed data processor adequate to execute the program components described herein and/or various specialized processing units as may be known in the art. In some examples, the one or more processors 208 may be a single processor, multiple processors, or multiple processor cores on a single die. In particular embodiments, the one or more processors 208 includes a graphical processing unit adequate for training one or more generative adversarial network models, one or more deep-learning-based networks, one or more neural networks, and/or the like.

In some examples, the interface bus 406 may include a network interface 412 configured to connect the device 400 to a communications network 414, an input/output ("I/O") interface 416 configured to connect and communicate with one or more peripheral devices, and/or a memory interface 418 configured to accept, communication, and/or connect to a number of machine-readable memory devices (e.g., memory 404).

The network interface 412 may operatively connect the device 400 to a communications network 414, which can include a direct interconnection, the Internet, a local area network ("LAN"), a metropolitan area network ("MAN"), a wide area network ("WAN"), a wired or Ethernet connection, a wireless connection, and similar types of communications networks, including combinations thereof. In some examples, device 400 may communicate with one or more remote / cloud-based servers 420 (e.g., database 304), cloud-based services 422, and/or remote devices via the communications network 414 and the network interface 412.

The memory 404 can be variously embodied in one or more forms of machine-accessible and machine-readable memory. In some examples, the memory 404 includes a storage device 424 comprises one or more types of memory. For example, the storage device 424 can include, but is not limited to, a non-transitory storage medium, a magnetic disk storage, an optical disk storage, an array of storage devices, a solid-state memory device, and the like, including combinations thereof.

Generally, the memory 404 is configured to store data / information 426 and instructions 428 that, when executed by the one or more processors 208, causes the device 400 to perform one or more tasks. In particular examples, the memory 404 includes a consent revocation package 430 that comprises a collection of program components, database components, and/or data. Depending on the particular implementation, the consent revocation package 430 may include software components, hardware components, and/or some combination of both hardware and software components.

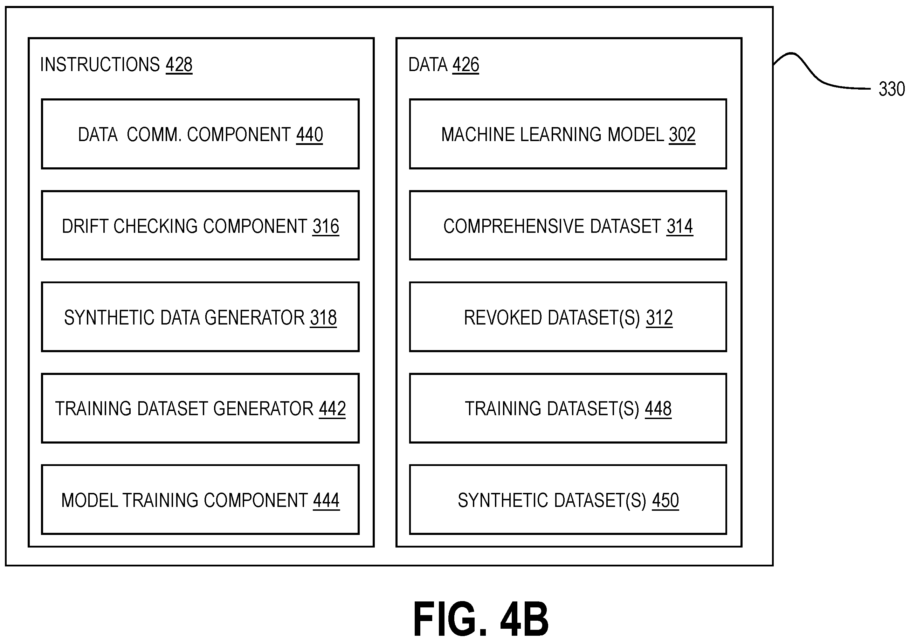

For example, with reference to FIG. 3 and FIG. 4B, the consent revocation package 430 may include, but is not limited to, instructions 428 having one or more software packages configured to perform one or more steps of the methods described herein. These software packages may be incorporated into, loaded from, loaded onto, or otherwise operatively available to and from the device 400. Put another way, the consent revocation package 430 and/or one or more software packages may be incorporated into, loaded from, loaded onto, or otherwise operatively available to the processors 208 of the system 300.

In particular embodiments, the consent revocation package 430 can include, but is not limited to, instructions 428 having a data communication component 440, a drift checking component 316, a synthetic data generator 318, and/or a model training component 444. These components may be incorporated into, loaded from, loaded onto, or otherwise operatively available to and from the one or more processors 208 of the system 300.

The data communication component 440 can be a stored program component that is executed by at least one processor, such as the one or more processors 208 of the system 300. In particular, the data communication component 440 can be configured to request and receive and/or otherwise access available information stored in a database, such as database 304, as described herein. In embodiments, the data communication component 440 can be configured to receive at least a comprehensive dataset 314 and/or one or more subsets of data containing data for which consent has been revoked. In embodiments, the data communication component 440 may communicate directly with the database 304 and/or indirectly through the consent management system 306.

The drift checking component 316 can be a stored program component that is executed by at least one processor, such as the one or more processors 208 of the system 300. In particular, the drift checking component 316 can be configured to estimate the impact of at least a first subset of data 312 on the machine learning model 302, as described herein.

The synthetic data generator 318 can be a stored program component that is executed by at least one processor, such as the one or more processors 208 of the system 300. In particular, the synthetic data generator 318 can be configured to generate one or more synthetic datasets 450 corresponding to at least the first subset of data 312, as described herein.

The training dataset generator 442 can be a stored program component that is executed by at least one processor, such as the one or more processors 208 of the system 300. In particular, the training dataset generator 442 can be configured to generate one or more training datasets 448 based on the information available from the comprehensive dataset 314 and the synthetic dataset(s) 450. Put another way, the training dataset generator 442 may be configured to generate one or more training datasets 448 comprising at least the comprehensive dataset 314 and one or more synthetic datasets 450, as described herein.

The model training component 444 can be a stored program component that is executed by at least one processor, such as the one or more processors 208 of the system 300. In particular, the model training component 444 can be configured to train the machine learning model 302 based on the training dataset(s) 448. In embodiments, the comprehensive dataset 314 used in the training datasets 448 does not include the revoked datasets 312. In other embodiments, the model training component 444 is also configured to train the machine learning model 302 based on the comprehensive dataset 314 prior to receiving one or more consent revocation notices such the machine learning model 302 is trained on a comprehensive dataset 314 containing data that is subsequently revoked (e.g., revoked instances 312).

The device 400 may also include an operating system component 432, which may be stored in the memory 404. The operating system component 1032 may be an executable program facilitating the operation of the device 400. Typically, the operating system component 432 can facilitate access of the I/O interface, network interface, and memory interface, and can communicate with other components of the system 300.

As described herein, the systems and method for training a machine learning model in a data environment where the training data is subject to deletion and/or quarantine provide increased stability to resultant machine learning models, enable resultant machine learning models to grasp insights from revoked data that would otherwise be missed.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory, a read-only memory, an erasable programmable read-only memory (EPROM or Flash memory), a static random access memory (SRAM), a portable compact disc read-only memory (CD-ROM), a digital versatile disk, a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network or a wide area network, or the connection can be made to an external computer (for example, through the Internet using an Internet Service Provider). In some examples, electronic circuitry comprising, for example, programmable logic circuitry, field-programmable gate arrays (FPGA), or programmable logic arrays can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) of training a machine learning model (302), the method (100) comprising:
receiving (110) a consent revocation notice, wherein the consent revocation notice identifies a first subset of data (312) from a comprehensive dataset (314);
estimating (120) an impact of the first subset of data (312) on the machine learning model (302);
generating (130) a synthetic dataset (450) corresponding to the first subset of data (312);
generating (140) a training dataset (448) comprising the comprehensive dataset (314) and the synthetic dataset (450); and
training (150) the machine learning model (302) on the training dataset (448).

2. The method (100) of claim 1, wherein the training dataset (448) does not include the first subset of data (312).

3. The method (100) of claim 1, wherein the consent revocation notice identifies the first subset of data (312) for which consent to use the first subset of data (312) has been revoked.

4. The method (100) of claim 1, further comprising:
training (105) the machine learning model (302) on the comprehensive dataset (314) before receiving a consent revocation notice, wherein the comprehensive dataset (314) comprises at least the first subset of data (312).

5. The method (100) of claim 1, wherein generating (130) the synthetic dataset includes:
generating (131) the synthetic dataset (450) using a generative adversarial network, wherein the synthetic dataset (450) has one or more data features;
determining (133) whether the one or more data features of the synthetic dataset (450) match one or more data features of the first subset of data (312) within a predetermined threshold; and
repeating (135) the generation (131) and determination (133) steps until the one or more data features of the synthetic dataset (350) match the one or more data features of the first subset of data (312) within the predetermined threshold.

6. The method (100) of claim 5, wherein the one or more features of the synthetic dataset (450) includes at least one of a dataset scale, a dataset shape, a dataset threshold, and a dataset distribution; and
wherein the one or more features of the first subset of data (312) includes at least one of a dataset scale, a dataset shape, a dataset threshold, and a dataset distribution.

7. The method (100) of claim 1, wherein the synthetic dataset (450) is generated in response to the estimated impact of the first subset of data (312) on the machine learning model (302).

8. The method (100) of claim 7, wherein the estimated impact comprises a count of one or more model parameters and/or predictions that are likely to change when the machine learning model (302) is trained using the training dataset (448) that does not include the first subset of data (312).

9. The method (100) of claim 7, wherein the estimated impact comprises a degree to which one or more model parameters and/or predictions are likely to change by when the machine learning model (302) is trained using the training dataset (448) that does not include the first subset of data (312).

10. A system (300) configured to train a machine learning model (302), the system (300) comprising:
a database (304) storing a comprehensive dataset (314) of medical information of a plurality of patients;
a consent management system (306) configured to manage consent settings for the medical information of the plurality of patients; and
one or more processors (308) in communication with the database (304) and the consent management system (306), the one or more processors being configured to:
receive a consent revocation notice from the consent management system (306), wherein the consent revocation notice identifies a first subset of data (312) from the comprehensive dataset (314);
estimate an impact of the first subset of data (312) on the machine learning model (302);
generate a synthetic dataset (450) corresponding to the first subset of data (312); and
generate a training dataset (448) comprising the comprehensive dataset (314) and the synthetic dataset (450); and
train the machine learning model (302) on the training dataset (448).

11. The system (300) of claim 10, wherein the training dataset (448) does not include the first subset of data (312).

12. The system (300) of claim 10, wherein the one or more processors (208) are further configured to:
train the machine learning model (302) on the comprehensive dataset (314) before receiving a consent revocation notice, wherein the comprehensive dataset (314) comprises at least the first subset of data (312).

13. The system (300) of claim 10, wherein the one or more processors (208) are configured to generate the synthetic dataset (450) by:
generating the synthetic dataset (450) using a generative adversarial network, wherein the synthetic dataset (450) has one or more data features;
determining whether the one or more data features of the synthetic dataset (450) matches one or more data features of the first subset of data (312) within a predetermined threshold; and
repeating the generation and determination steps until the one or more data features of the synthetic dataset (450) matches the one or more data features of the first subset of data (312) within the predetermined threshold.

14. The system (300) of claim 13, wherein the one or more features of the synthetic dataset (450) includes at least one of a dataset scale, a dataset shape, a dataset threshold, and a dataset distribution; and
wherein the one or more features of the first subset of data (312) includes at least one of a dataset scale, a dataset shape, a dataset threshold, and a dataset distribution.

15. The system (300) of claim 10, wherein the synthetic dataset (450) is generated in response to the estimated impact of the first subset of data (312) on the machine learning model (302).
